# EUROPEAN PATENT APPLICATION

(11) **EP 3 738 435 A1**
(43) Date of publication of application: **18.11.2020**
(21) Application number: 20166825.8
(22) Date of filing: 30.03.2020
(51) Int. Cl.: A21D 2/20, A61K 8/73

(54) **PROCESS FOR EXTRACTING GLUCO-OLIGOSACCHARIDE FROM THE BABASSU MESOCARP FLOUR FROM ORBIGNYA PHALERATA, GLUCO-OLIGOSACCHARIDE EXTRACTED BY SAID PROCESS AND USE OF THE GLUCO-OLIGOSACCHARIDE**

(30) Priority: 01.04.2019 BR 102019006616
(71) Applicant: Atina Industria e Comercio de Ativos Naturais Ltda, 37550-000 Pouso Alegre - MG (BR)
(72) Inventor: COELHO BENEVIDES, Paulo José, 05409-011 Praia Grande - SP (BR); LICHTENSTEIN DE MORARES, Márcia, 04747-030 Santo Amaro - SP (BR)
(74) Representative: Winter, Brandl, Fürniss, Hübner, Röss, Kaiser, Polte - Partnerschaft mbB

(57) **Abstract**

The present invention refers to a process for extracting a white and highly crystalline gluco-oligosaccharide from the crude flour of the mesocarp of the fruits of palm trees of the genus *Orbignya* spp. Further, the present invention relates to a white gluco-oligosaccharide with high crystallinity and high Amylopectin content extracted by said process for use in the cosmetic, pharmaceutical, dermocosmetic and nutraceutical areas, and the extracted gluco-oligosaccharide of the present invention comprises a high oil adsorption capacity and, by promoting a matte effect, in addition to its white color, it does not influence the final color of the product being applied.

## Description

### Field of the invention

The present invention refers to a process for extracting a white and highly crystalline gluco-oligosaccharide from the crude flour of the mesocarp of the fruits of palm trees of the genus *Orbignya* spp. Further, the present invention relates to a white gluco-oligosaccharide with high crystallinity and high Amylopectin content extracted by said process for use in the cosmetic, pharmaceutical, dermocosmetic and nutraceutical areas, and the extracted gluco-oligosaccharide of the present invention comprises a high oil adsorption capacity and, by promoting a matte effect, in addition to its white color, it does not influence the final color of the product being applied.

### Background of the Invention

Among the species of palm trees used in the Brazilian extractive industry, babassu stands out for its economic potential and multiple uses. Oleaginous palm tree found in the Amazon Forest, in the Cerrado and in the transition zone between these biomes, belongs to the genus *Orbignya,* which has the genus *Attalea* as a synonym and is divided into at least 5 species whose areas of occurrence partially overlap.

The babassu palm can reach up to 20 meters in height and it can be exploited from the root to the leaves. The raw material used in the manufacture of margarine, soap and cosmetics is extracted from this species. The sprout of this palm provides good quality hearts of palm, and the fruit, while green, serves to smoke rubber, and when ripe, its external part is edible.

The Cocais forests, dense agglomerations of babassu existing between Caatinga, Cerrado and Amazonia, occupy about 18 million hectares of central Brazil. Babassu has great potential for generating wealth for collector communities, based on its various uses. Throughout this region, there are municipal laws of Babaçu Livre (Free Babassu), which ensure the access of collectors to palm trees located in public and private areas.

There are several species of babassu, including *Orbignya phalerata,* whose mesocarp is extracted and transformed into babassu flour by structured communities in Maranhão and southern Pará. The harvest of babassu occurs at the end of the drought period in the Amazon.

Babassu coconut consists of three layers, the fibrous outer layer (epicarp); the intermediate, fibrous-starchy (mesocarp); and the internal, woody (endocarp), in which the almonds are inserted. The set of three layers - epicarp, mesocarp and endocarp - is usually called shell, corresponding to approximately 93% of the total coconut. Babassu palms produce up to 5 bunches per crop, with about 400 coconuts per bunch. Coconuts weigh up to 300 grams, 20% of which is mesocarp.

The mesocarp, when fresh, has a light cream color and can easily be reduced to powder. As it ages, it acquires woody stiffness and a reddish-brown color. When dry, after soaking in water, it has a texture similar to latex, being difficultly ground or crushed.

Babassu coconut flour is generally used in its raw form without any processing. The only existing processes for isolating polysaccharides from this flour are those described below, which, however, do not produce a high crystallinity gluco-oligosaccharide (high amylopectin content).

The Brazilian patent application BR 11 2014 004854 1, filed on August 31, 2011, under the title:"Composição cosmética destinada a maquiagem da pele, produto cosmético e uso cosmético de polissacarídeos de babaçu [Cosmetic composition for skin make-up, cosmetic product and cosmetic use of babassu polysaccharides], on behalf of Natura Cosméticos S.A., describes a cosmetic composition intended for skin make-up that comprises: (a) a sensory modifying system comprising at least babassu polysaccharide; (b) at least one treated pigment; and (c) at least one optical diffuser. This cosmetic composition covers the skin evenly, presenting full coverage of the skin region, good adhesion, minimizes excessive shine, among other advantages. The invention also refers to the cosmetic use of babassu polysaccharide as a sensory modifier. In this case, babassu flour is applied to cosmetics in its natural color, which narrows its applicability in the cosmetic industry.

Brazilian patent application No. PI 0904836-7, filed on December 22, 2009, under the title: "Composições farmacêuticas, processo de obtenção de composições farmacêuticas e uso de composição farmacêutica a base do mesocarpo do fruto de *Orbignya* phalerata"[Pharmaceutical compositions, process for obtaining pharmaceutical compositions and use of pharmaceutical composition based on the mesocarp from the fruit of *Orbignya phalerata*], on behalf of Universidade Federal Do Maranhão, describes a process for obtaining the alcoholic extract of the mesocarp from the fruits of *Orbignya phalerata,* having pharmacodynamic activity, pharmaceutical compositions characterized by containing from 0.005mg to 50000mg of mesocarp or alcoholic extract obtained from the mesocarp of the fruits of *Orbignya phalerata* and other pharmacodynamically active components, therapeutic use of the compositions in the prevention and treatment of inflammations, infections, and as an aid in the tissue healing process. The invention aims to formulate cosmetic and pharmaceutical compositions (solution, cream, gel, capsules, etc.) with anti-inflammatory, healing and antimicrobial activity, obtained from the aqueous and/or ethanolic extract of the mesocarp from the fruit of *Orbignya phalerata,* disregarding the starchy composition of babassu flour.

European patent EP 1584332 B1, filed on September 29, 2001, under the title: "*Process for bleaching natural substances*", on behalf of Peter GrevenFett-Chemie GMBH & CO.KG, describes a process for bleaching flour made from natural products, comprising mixing flour with water and, optionally, additives, adding hydrogen peroxide and an alkali metal hydroxide. When the pH of the mixture is reduced to a value of 10 or less, an acid is added until hydrogen peroxide can no longer be detected, followed by the addition of other additives to the mixture. The invention is not amenable to the bleaching treatment of babassu mesocarp flour since it forms latex/gel under the conditions of the invention, resulting in loss of particle integrity, especially the starch combination.

US patent No. 6,497,909, filed on September 9, 1999, under the title: *"Method of bleaching cereal grain*", in the name of General Mills, Inc., describes bleached cereal products, such as bleached whole wheat flour, which are obtained with the color and flavor of white flour by bleaching whole wheat kernels before conventional milling. The bleached grain kernels can be seasoned and ground into whole grain flours that have 10% to 12% dietary fiber. Wheat seeds are treated with a hydrogen peroxide solution to lighten the color of the bran layers, also involving the addition of an alkaline solution. The invention declares the bleaching of grains in aqueous solution before the milling process for conversion to flour. In addition, the inventive process causes, if applied to babassu flour, the formation of latex/gel, resulting in the loss of integrity of the particles, especially the starch combination.

Brazilian patent application No. BR 10 2015 025923 9, filed on October 9, 2015, under the title: "PROCESSO PARA BRANQUEAMENTO DA FARINHA DO MESOCARPO DO BABAÇU ORBIGNYA spp, E USO DA FARINHA DE BABAÇU BRANQUEADA PRODUZIDA" [PROCESS FOR BLEACHING THE BABASSU MESOCARP FLOUR FROM ORBIGNYA spp, AND USE OF PRODUCED BLEACHED BABASSU FLOUR], on behalf of ATINA INDUSTRIA E COMERCIO DE ATIVOS NATURAIS LTDA describes a process for bleaching the flour from the mesocarp of *Orbignya phalerata,* by extracting and processing into white babassu flour. Thus, it is observed that the document does not describe a process for extracting a white gluco-oligosaccharide, with high crystallinity and high amylopectin content from the flour of the mesocarp of *Orbignya phalerata.*

Thus, as can be seen, no state-of-the-art document describes or suggests a process for extracting a high-crystalline, high-amylopectin content and white-colored gluco-oligosaccharide from babassu mesocarp flour of *Orbigya phalerata,* and the process of the present invention provides ideal conditions for the extraction of a gluco-oligosaccharide with the mentioned characteristics.

### Summary of the Invention

It is an object of the present invention to provide a process for extracting a gluco-oligosaccharide from the mesocarp flour of *Orbignya phalerata,* comprising the steps of:
a) pre-processing babassu fruits to extract crude flour from babassu mesocarp, comprising the steps of:
   a.1) collecting 90% of the existing babassu fruits and keeping the other 10%, of good quality, in the area for natural regeneration;
   a.2) transporting the babassu fruits on the same day of collection to the processing shed;
   a.3) performing, in the processing unit, the second selection of babassu fruits;
   a.4) washing the babassu fruits, before peeling;
   a.5) removing the mesocarp from the babassu fruit in the form of flakes (flakes);
   a.6) passing the flakes through lathe or beat with a mallet in a basin or bucket;
   a.7) drying the flakes;
   a.8) crushing the dry flake to form the babassu mesocarp crude flour;
   a.9) sieving the babassu mesocarp crude flour; and
   a.10) packing the sieved crude flour;
      after pre-processing to prepare the crude flour,
b) grinding the babassu mesocarp crude flour prepared in the aforementioned step a) in a hammer mill, to reduce the mesocarp in a regular grain-sized flour;
c) in a reactor, bleaching the obtained babassu mesocarp flour, adding 1 part of the ground babassu flour, with 1 to 15 parts of 96°GL ethyl alcohol;
d) stirring the mixture for 20 to 30 minutes for complete homogenization;
e) making the suspension alkaline with the addition of 1 to 10 parts of sodium hydroxide solution under constant agitation, avoiding the spilling of the solution on the reactor walls;
f) stirring for 25 to 30 minutes, until complete homogenization.
g) adding 1 to 30 parts of hydrogen peroxide, stirring slowly, until all the plant material has been bleached;
h) washing with purified water until the hydrogen peroxide residue is completely removed;
i) extracting from the said suspension the gluco-oligosaccharide obtained by centrifugation.

In addition, the present invention describes the gluco-oligosaccharide obtained by said process comprising white color, Amylopectin content between 60% to 90% and degree of crystallinity between 30% and 36%.

Furthermore, the present invention describes the use of gluco-oligosaccharide extracted from the babassu mesocarp flour of *Orbignya phalerata* by said process, in cosmetic and pharmaceutical formulations, particularly in emulsions, sunscreen, chafing cream, aftershave lotion, aftershower cream, makeup and/or pressed or loose powders, as well as in a cosmetic formulation of sunscreen as an oil absorber and in pharmaceutical products as a sensory-modifying excipient.

### Brief Description of the Drawings

Figure 1 shows the overlap of diffractograms obtained under similar conditions of analysis for the Amylopectin standard samples and the obtained gluco-oligosaccharide samples.

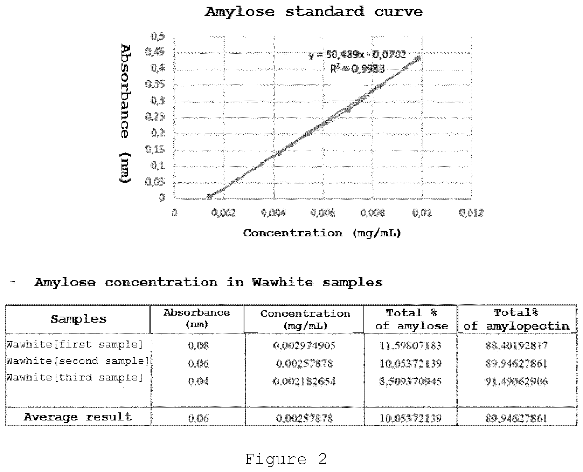

Figure 2 shows the Amylose standard curve constructed to determine, by the colorimetric method, the Amylopectin content (by difference) in the samples of high crystallinity gluco-oligosaccharide obtained.
Figure 3 presents a comparative graph of the functional attributes in SPF 30 sunscreen formulas.

### Detailed Description of the Invention

While the present invention may be susceptible to different embodiments, it is shown in the drawings and the following detailed discussion, preferred embodiments with the understanding that the present disclosure should be considered an exemplification of the principles of the invention and is not intended to limit the present invention to what was illustrated and described in this disclosure.

The main approach of this invention is related to a process for obtaining a gluco-oligosaccharide from the flour of the mesocarp of *Orbignya phalerata,* comprising the steps of:
a) pre-processing babassu fruits to extract crude flour from babassu mesocarp, comprising the steps of:
   a.1) collecting 90% of the existing babassu fruits and keeping the other 10%, of good quality, in the area for natural regeneration;
   a.2) transporting the babassu fruits on the same day of collection to the processing shed;
   a.3) performing, in the processing unit, the second selection of babassu fruits;
   a.4) washing the babassu fruits, before peeling;
   a.5) removing the mesocarp from the babassu fruit in the form of flakes (flakes);
   a.6) passing the flakes through lathe or beat with a mallet in a basin or bucket;
   a.7) drying the flakes;
   a.8) crushing the dry flake to form the babassu mesocarp crude flour;
   a.9) sieving the babassu mesocarp crude flour; and
   a.10) packing the sieved crude flour;
      after pre-processing to prepare the crude flour,
b) grinding the babassu mesocarp crude flour prepared in the aforementioned step a) in a hammer mill, to reduce the mesocarp in a regular grain-sized flour;
c) in a reactor, bleaching the obtained babassu mesocarp flour, adding 1 part of the ground babassu flour, with 1 to 15 parts of 96°GL ethyl alcohol;
d) stirring the mixture for 20 to 30 minutes for complete homogenization;
e) making the suspension alkaline with the addition of 1 to 10 parts of sodium hydroxide solution under constant agitation, avoiding the spilling of the solution on the reactor walls;
f) stirring for 25 to 30 minutes, until complete homogenization;
g) adding 1 to 30 parts of hydrogen peroxide, stirring slowly, until all the plant material has been bleached;
h) washing with purified water until the hydrogen peroxide residue is completely removed;
i) extracting from the said suspension the gluco-oligosaccharide obtained by centrifugation.

The process for extracting the gluco-oligosaccharide of the present invention provides a white crystalline gluco-oligosaccharide with high crystallinity and high amylopectin content, as its steps for pre-processing babassu fruits provide a crude mesocarp flour from *Orbignya phalerata* in ideal conditions for such extraction.

Thus, the present invention also describes the gluco-oligosaccharide obtained by said process comprising white color, Amylopectin content between 60% to 90%, preferably between 70% and 90% and a degree of crystallinity between 30% and 36%.

The white gluco-oligosaccharide, with high crystallinity of the amylopectin content of the present invention has the following advantages and benefits:
- product of renewable, vegetal, traceable and sustainable origin;
- high oil absorption capacity of formulations;
- promotes matte effect and presents pleasant sensory properties;
- improves sliding in compacted, molded and loose powder products;
- viscosity modulator in cosmetic, dermocosmetic and pharmaceutical galenic forms;
- excellent performance in hydro-alcoholic systems;
- applicability to a wider range of cosmetic, dermocosmetic and pharmaceutical products without interfering with its color.

With respect to the process of the present invention, the collection from step a.1) is performed with the aid of babassu fruit sticks.

In step a.2), babassu fruits for mesocarp extraction must be processed within 15 days of the harvest date in the field and must remain stored in a shed with natural ventilation and raised from the ground, in the case of beetle infestation.

In step a.4), babassu fruits must be washed, before peeling by immersion in a tank with chlorinated water and washed with the aid of a brush and peeled soap to extract the mesocarps in the form of flakes; then the flakes must be passed through lathe or beaten with a mallet in a basin or bucket (put on a high lined and covered table to protect from insects and dust).

In step a.7), drying is performed in an oven at a temperature between 70 and 90°C. Optionally, the flakes can dry in the sun, spread on top of a clean plastic, in an environment protected from insects and dust.

In said step a.8), the process of crushing the dry flake can be performed in the pestle, in the fodder machine or in the mill, in this case, it is recommended that the mill is made of stainless steel.

In the sieving from step a.9), the dry and crushed flour (crude babassu mesocarp flour) must be sieved to remove lint from the shell, black spots and other impurities.

In step a.10) of the aforementioned process, the packing is done in plastic bags, and the bags must be kept in dry, clean, fumigated and airy places.

In this process, 96°GL ethyl alcohol is used instead of an aqueous solution, due to the reaction temperature reaching around 75°C, which would form an amylaceous gel in an aqueous medium, changing the particle shape and, consequently, the sensory properties of said raw material.

The reactor in step c) must be made of stainless steel with a vacuum system to reduce the internal pressure caused by the release of nascent oxygen during the bleaching process with hydrogen peroxide.

Also, in step e), the alkali metal hydroxide solution used is preferably 50% NaOH solution. Alkali metal hydroxide is the catalyst for the oxidation reaction of the remaining tannins. Its addition must be performed slowly to avoid agglutination of solid and rapid formation of nascent oxygen ([O]).

Steps (g), (h) and (i) are repeated 2 more times in order to guarantee the high crystallinity of the derived gluco-oligosaccharide. If the bleaching is not satisfactory, or if the required crystallinity is not achieved, the addition of hydrogen peroxide and catalyst should be repeated as many times as necessary until reaching the desired specification.

In said step i) the extraction of the gluco-oligosaccharide is performed in a centrifugal extractor-type industrial centrifuge type made of stainless steel, automatic discharge from the bottom.

Optionally, said step i) can be carried out with the aid of a filter press with a filter element with mesh between 10 and 2 µm, in which case, the retained material must be rinsed in the filter elements with purified water solution and the solution extracted must be spray dried.

After the extraction of the gluco-oligosaccharide from the crude babassu mesocarp flour, the determination of the amylopectin content and of the crystallinity of the gluco-oligosaccharide must be performed.

The determination of the Amylopectin content and the crystallinity of the gluco-oligosaccharide is performed by X-ray diffractometry (XRD) of polycrystalline materials (powders) to estimate the degree of crystallinity of the sample and to qualitatively and semi-quantitatively evaluate the presence of Amylopectin in each of them. This technique consists of detecting X-rays diffracted by a material due to the interaction of incident radiation with the atoms in the sample. The diffracted radiation is measured as a function of the Bragg angle and the presence of crystalline phase is indicated by Bragg reflections with specific intensity and angle for many crystalline planes.

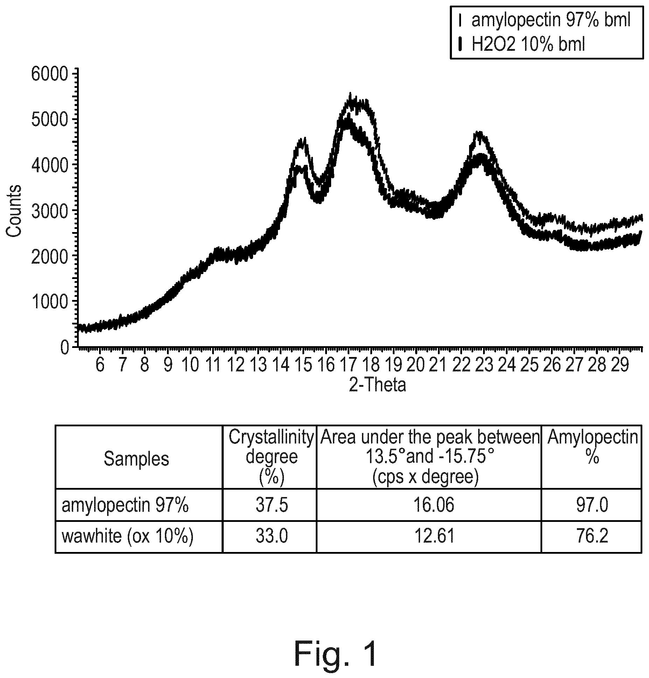

Figure 1 shows the overlap of diffractograms obtained under similar conditions of analysis for the Amylopectin standard samples and the obtained gluco-oligosaccharide samples. The degree of crystallinity of each sample was assessed by measuring the total area under the curve. The Amylopectin content was obtained from the same diffractogram, first adjusting the background, and integrating the areas of the sample signals in the range of angles 13.5° and 15.75° (2θ). The integration of the area was evaluated by the Diffrac.Eva software.

Optionally, the determination of the Amylopectin content can be performed indirectly through the determination of Amylose (since starch is a homopolysaccharide composed only by Amylose and Amylopectin) by the colorimetric method, as described by Williams et *al.* (WILLIAMS, P. C.; KUZINA, F. D.; HLYNKA, I. A. Rapid colorimetric procedure for estimating the amylase content of starch on fluors. Cereal Chemistry, v. 47. n. 4. p. 412-420, 1970), with adjustments.

The colorimetric methodology, previously described, is used as a process control since the determination can be made in quality control laboratories using spectrophotometer. However, the XRD methodology is more accurate and should be used to confirm and guarantee the specified crystallinity range.

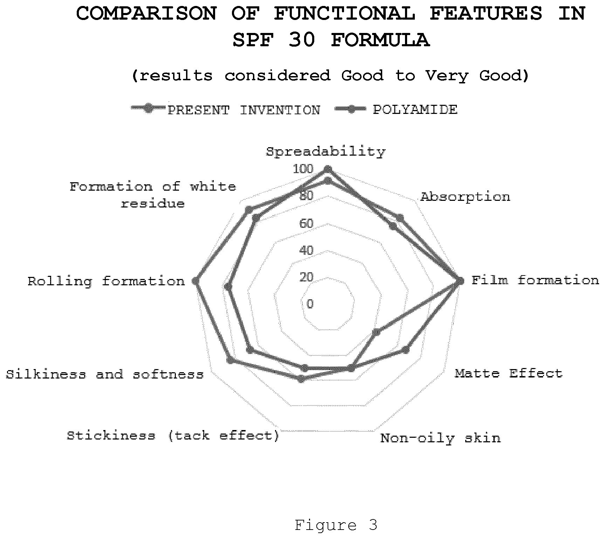

Figure 3 shows the Amylose standard curve constructed to determine the Amylopectin content (by difference) in the samples of high crystallinity gluco-oligosaccharide obtained by the colorimetric method.

The white and highly crystalline gluco-oligosaccharide produced by the process described above has the following specifications and characteristics:

| | |
|---|---|
| Mean diameter: | 10µm |
| Max diameter: | 30µm |
| Humidity: | < 6% |
| Density: | 0.5 - 0.7 g/cm² |
| pH: | 5.0 - 7.0 |
| Amylopectin content: | 60 - 90 % |
| Crystallinity Degree: | 30 - 36% |
| Bacterial count: | < 100 CFU/g |
| Fungi Count: | < 100 CFU/g |
| Pathogens: | absent |

100g of product adsorb 54g of flaxseed oil

In addition, the present invention describes the use of gluco-oligosaccharide extracted by said process as a feedstock for different cosmetic formulations for the face and body as an oil-absorbing agent. The application of the white and highly crystalline gluco-oligosaccharide extracted occurs mainly in the cosmetic, dermocosmetic and pharmaceutical field, particularly in emulsions, sunscreen, chafing cream, aftershave lotion, aftershower cream, makeup and/or pressed or loose powders, as well as in a cosmetic formulation of sunscreen as an oil absorber and in pharmaceutical products as a sensory-modifying excipient.

### Example 1

An example of these uses of the gluco-oligosaccharide of the present invention is the FPS 30 formula and the results of tests with volunteers, performed in an external institute, comparing the same cosmetic formula with different commercial fillers in the same application concentration.

A SPF 30 sunscreen formulation, containing 1% of the raw material was evaluated in a sensory test with 12 volunteers. The results clearly showed that the raw material, gluco-oligosaccharide, presents similar results in the sensory evaluation, and in some parameters superior to commercial fillers available on the market.

Gluco-oligosaccharide obtained from babassu mesocarp flour was applied in a cosmetic formulation of an SPF 30 sunscreen, containing a high load of chemical filters, which naturally give an oily sensory property, in the proportion of 1% as well as commercial fillers for comparison of performance in the final formula. The cosmetic formulation of a SPF 30 sunscreen is shown in Table 1 below.

**Table 1 - Cosmetic formulation of sunscreen - SPF 30**

| Raw material | % |
|---|---|
| Deionized water | q.s. 100 |
| Disodium EDTA | 0.1 |
| Hydroxyacetophenone | 0.5 |
| Acrylic acid/VP Cross-polymer | 0.4 |
| Sodium hydroxide | 0.04 |
| Avobenzone | 3.5 |
| Octyl salicylate | 3 |
| Octocrylene | 7 |
| Glycerol Stearate (and) Laureth-23 | 1.5 |
| Tridecyl Neopentanoate | 3 |
| VP/Eicosene Copolymer | 2 |
| Titanium dioxide/Phenethyl benzoate/Isocetyl stearoyl stearate | 5 |
| Bis-Ethylhexyloxyphenol methoxyphenyl triazine | 3.3 |
| Homosalate | 9.5 |
| Potassium Cetyl Phosphate | 1.5 |
| Sodium Hydroxide | 0.25 or pH 6.2-7.2 |
| Denatured ethyl alcohol | 1.5 |
| Gluco-oligosaccharide of babassu mesocarp flour | 1 |
| Glycerin | 1.5 |
| 1,2-Hexanediol and Caprylyl glycol | 0.5 |

Sensory evaluation of white and highly crystalline gluco-oligosaccharide flour in a cosmetic formula for sun protection.

The study was planned and conducted by determination of Resolution No. 466/12 of the *National Health Council on Regulating Guidelines and Standards for Research Involving Humans.*

12 volunteers completed the study: mean age 27 +/- 6 years, phototype (Fitzpatrick) 50% phototype III and 50% phototype IV, where no adverse reactions were reported or presented during the study.

The following parameters were evaluated as mentioned in Table 2 below:

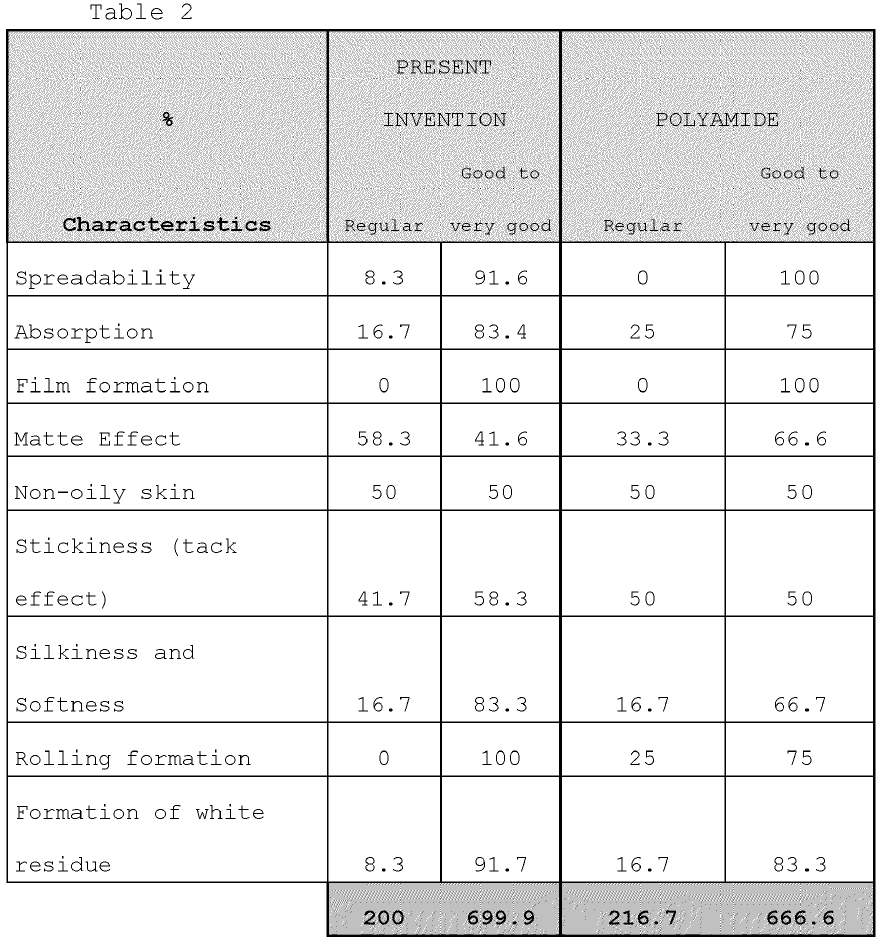

Thus, although only some embodiments of the present invention have been shown, it will be understood that various omissions, substitutions and changes can be made by a person skilled in the art, without departing from the spirit and scope of the present invention. The described embodiments should be considered in all aspects only as illustrative and not restrictive.

It is expressly provided that all combinations of elements that perform the same function substantially in the same way to achieve the same results are within the scope of the invention. Substitutions of elements from one described embodiment to another are also fully intended and contemplated.

One must also understand that the drawings are not necessarily scaled, but that they are only conceptual in nature. The intent is therefore to be limited as indicated by the scope of the appended claims.

## Claims

1. A process for extracting a gluco-oligosaccharide from the flour of the mesocarp of *Orbignya phalerata,* **characterized by** comprising the steps of:
a) pre-processing babassu fruits to extract crude flour from babassu mesocarp, comprising the steps of:
a.1) collecting 90% of the existing babassu fruits and keeping the other 10%, of good quality, in the area for natural regeneration;
a.2) transporting the babassu fruits on the same day of collection to the processing shed;
a.3) performing, in the processing unit, the second selection of babassu fruits;
a.4) washing the babassu fruits, before peeling;
a.5) removing the mesocarp from the babassu fruit in the form of flakes;
a.6) passing the flakes through lathe or beat with a mallet in a basin or bucket;
a.7) drying the flakes;
a.8) crushing the dry flake to form the babassu mesocarp crude flour;
a.9) sieving the babassu mesocarp crude flour; and
a.10) packing the sieved crude flour;
after pre-processing to prepare the crude flour,
b) grinding the babassu mesocarp crude flour prepared in the aforementioned step a) in a hammer mill, to reduce the mesocarp in a regular grain-sized flour;
c) in a reactor, bleaching the obtained babassu mesocarp flour, adding 1 part of the ground babassu flour, with 1 to 15 parts of 96°GL ethyl alcohol;
d) stirring the mixture for 20 to 30 minutes for complete homogenization;
e) making the suspension alkaline with the addition of 1 to 10 parts of sodium hydroxide solution under constant agitation, avoiding the spilling of the solution on the reactor walls;
f) stirring for 25 to 30 minutes, until complete homogenization;
g) adding 1 to 30 parts of hydrogen peroxide, stirring slowly, until all the plant material has been bleached;
h) washing with purified water until the hydrogen peroxide residue is completely removed;
i) extracting from the said suspension the gluco-oligosaccharide obtained by centrifugation.

2. The process, according to claim 1, **characterized in that** the collection from step a.1) is performed with the aid of babassu fruit sticks.

3. The process, according to claim 1, **characterized in that,** in step a.2), babassu fruits for mesocarp extraction must be processed within 15 days of the harvest date in the field and must remain stored in a shed with natural ventilation and raised from the ground, in the case of beetle infestation.

4. The process, according to claim 1, **characterized in that,** in step a.4), the babassu fruits are immersed in a tank with chlorinated water and washed with the aid of a brush and peeled soap to extract the extracted flakes.

5. The process, according to claim 1, **characterized in that,** in step a.7), drying is done in an oven at a temperature between 70 and 90°C, and the flakes can also dry in the sun, spread on top of a clean plastic, in an environment protected from insects and dust.

6. The process, according to claim 1, **characterized in that,** in step a.8), the crushing can be performed in the pestle, in the fodder machine or in the mill.

7. The process, according to claim 1, **characterized in that** the step a.9) removes lint from the shell, black spots and other impurities.

8. The process, according to claim 1, **characterized in that** in step a.10) the packing is done in plastic bags, and the bags must be kept in dry, clean, fumigated and airy places.

9. The process, according to claim 1, **characterized in that** the reactor from step c) must be made of stainless steel with a vacuum system and/or
in said step e), the alkali metal hydroxide solution is preferably 50% and/or
the steps g), h) and i) are repeated 2 more times and/or
and/or the Amylopectin content and the crystallinity degree of the gluco-oligosaccharide extracted is determined by using the X-ray diffractometry (XRD) of polycrystalline materials to estimate the degree of crystallinity of the sample and to qualitatively and semi-quantitatively evaluate the presence of Amylopectin in each of them and/or
the determination of the Amylopectin content can also be performed indirectly through the determination of Amylose and/or
said step i) is performed in a stainless-steel centrifugal extractor.

10. The process, according to claim 1, **characterized in that** said step i) can also be performed in a filter press with a filter element with a mesh between 10 and 2 µm, with the material retained in the filter press being rinsed with purified water solution and spray dried.

11. The gluco-oligosaccharide obtained by the process as defined in claims 1 to 10, **characterized by** comprising white color, Amylopectin content between 60% to 90% and degree of crystallinity between 30% and 36%.

12. The gluco-oligosaccharide according to claim 11, **characterized in that** the Amylopectin content is preferably between 70% and 90%.

13. Use of the gluco-oligosaccharide as defined in claims 11 and 12, **characterized in that** it is in the cosmetic and pharmaceutical fields.

14. Use of the gluco-oligosaccharide, according to claim 13, **characterized in that** it is in emulsions, sunscreen, chafing cream, aftershave lotion, aftershower cream, makeup and/or pressed or loose powders.

15. Use of the gluco-oligosaccharide, according to claims 13 and 14, **characterized in that** it is in a cosmetic formulation of sunscreen as an oil absorber and in pharmaceutical products as a sensory-modifying excipient.
